Europäisches Patentamt

European Patent Office

Office européen des brevets

(19)

(11) Publication number: **0 404 478**
**A1**

# EUROPEAN PATENT APPLICATION

(21) Application number: **90306611.6**

(51) Int. Cl.5: **A61F 2/16**

(22) Date of filing: **18.06.90**

(30) Priority: **19.06.89 US 368252**
     **23.04.90 US 503249**

(43) Date of publication of application:
    **27.12.90 Bulletin 90/52**

(84) Designated Contracting States:
    **AT BE CH DE ES FR GB IT LI LU NL SE**

(71) Applicant: **IOPTEX RESEARCH INC.**
    **15715 Arrow Highway**
    **Irwindale, California 91706-2094(US)**

(72) Inventor: **Hunkeler, John D.**
    **5326 Mission Woods Road**
    **Shawnee Mission, KS 66205(US)**
    Inventor: **O'Hara, Karen**
    **1017 Bonita Avenue**
    **La Verne, CA 91750(US)**

(74) Representative: **Cole, William Gwyn, Dr.**
    **Smith & Nephew p l c Corporate Patents and**
    **Trade Marks Dept. Gilston Park**
    **Harlow Essex CM20 2RQ(GB)**

(54) Single piece ovoid introcular lenses with haptics.

(57) An intraocular lens (10) for implantation in the eye comprises an ovoid lens having upper and lower haptics (11) integral therewith. The haptics are of substantially equal stiffness, and extend at an angle of from 0° to 15° as measured from the perpendicular to the optical axis of the lens, but do not extend beyond the shortest dimension of the lens optic.

EP 0 404 478 A1

EP 0 404 478 A1

## SINGLE PIECE OVOID INTRAOCULAR LENSES WITH HAPTIC

The present invention is concerned with intraocular lenses (IOLs) which are oval in shape and have haptics integral therewith.

Oval lenses are known in the prior art. For example, US Patent No. 4298994 describes an oval lens having two haptics one of an open loop configuration and the other of a closed loop configuration, the haptic of open configuration having a stiffness greater than the haptic of closed loop configuration. Those lenses, however, have haptics which are secured to the lens as by drilling holes in the lens and suitably securing the haptics to the lens by affixing one end in the case of the open loop configuration and both ends in the case of the closed loop configuration into the lens body. This has the disadvantage of reducing the usable lens area and when positioning bores such as those described in US Patent No. 4292994 as 30 and 32 are constructed, this further reduces the usable lens area.

The present invention provides an intraocular lens for implantation in the eye which comprises an ovoid lens having two haptics integral therewith, said haptics being of substantially equal stiffness, and wherein each haptic has an angle of from 0° to 15° as measured from the perpendicular to the optical axis of the lens.

The present invention further comprises an intraocular lens for implantation in the eye which comprises a transparent ovoid lens having two haptics portions formed integrally therewith, and said haptics portions being of substantially equal stiffness, and extending at an angle of from 0° to 15° as measured from the perpendicular to the optical axis of the lens and wherein the haptics portions do not extend beyond the shortest diameter of the lens.

Preferably the lens ie. the lens optic and haptics are made of poly methyl methacrylate (PMMA). Each haptic is aptly of an open loop configuration and is preferably of a modified C configuration. The lenses of the present invention are generally referred to as narrow profile lenses which means the shorter dimension or diameter of the optic is aptly less than 6mm and preferably about 5mm.

The haptics of the IOLs of the invention are of substantially equal stiffness and suitably are of substantially equal size and shape. The intraocular lenses of the invention are vaulted and have an angle of from 0 to 15° as measured from the perpendicular to the optical axis of the lens. Preferably, the angle is about 10°.

The ovoid lens portion of the intraocular lens may have a planar surface and a convex surface. Aptly the convex surface will form the upper surface of the lens and the haptics will have an angle of from 0 to 15° preferably about 10° as measured upwardly from the perpendicular to the optical axis of the lens.

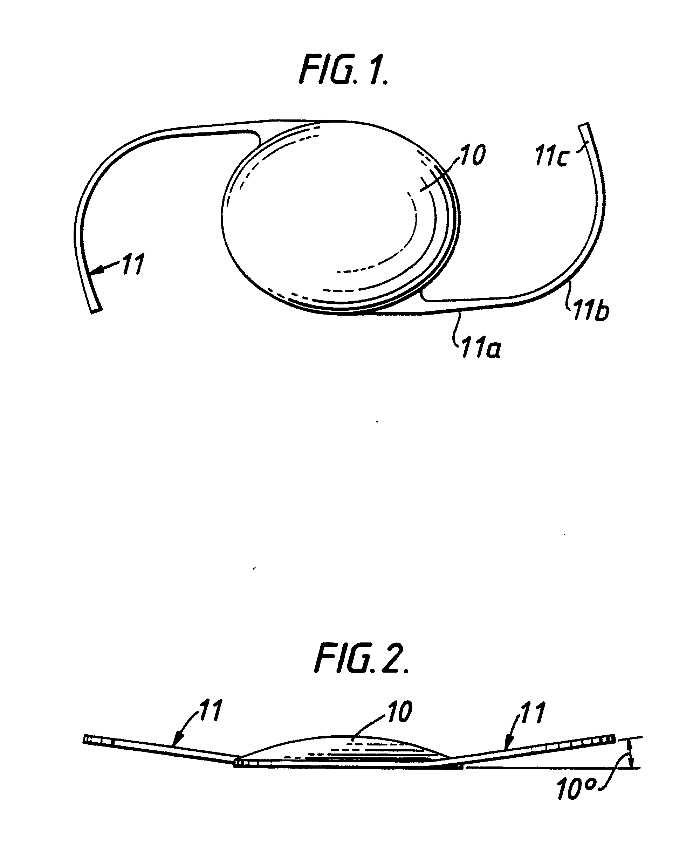

As shown in the drawings, Fig. 1 shows an oval lens optic 10 according to the present invention which may aptly measure approximately 6mm by 5mm. Integral with lens 10 are two haptics 11.

According to one embodiment of the present invention the haptics are in an arc configuration as shown in Fig. 1 and according to a further embodiment the arc is made up of three portions of unequal length. These portions are designated 11a, 11b and 11c. Preferably section 11a is substantially columnar and extends from the lens periphery from the shortest diametric point ie. the point where a line representing the shortest diameter or dimension of the ovoid lens intersects the circumference of the lens. Section 11a extends away from the lens periphery along a line which is substantially tangential to its point of intersection with the lens periphery. In alternative embodiments section 11a may extend from any point on the lens periphery, preferably tangentially from that point, provided that it does not extend outwardly beyond the shortest diameter of the lens. The proximal end of section 11a may be thickened at its intersection with the lens periphery.

Section 11b extends from section 11a in arcuate manner following generally the curvature of the ovoid lens periphery. Preferably the degree of curvature is greater than that of the lens so that the second portion 11b curves outwardly from the lens centre.

Section 11c also is arcuate and follows generally the curvature of the periphery of the lens. However, it is preferred that the degree of curvature is less than that of section 11b. Section 11c may terminate at its distal end with a thickened portion to provide a suitable abutment to the eye tissue when the intraocular lens is in place. The end of section 11c does not extend beyond the width of the narrowest portion of the lens optic.

In accordance with this embodiment as shown in the drawings there is therefore provided an intraocular lens for implantation in the eye which comprises one convex-plano ovoid lens having two haptics integral therewith, said haptics being of substantial equal stiffness wherein each of said haptics comprises a first substantially columnar portion extending substantially tangentially away from the shortest diametric point on the lens periphery, a second arcuate portion and a third arcuate portion whose curvature is less than that of

2

the second portion, wherein each haptic has an angle of from 0° to 15° as measured from the perpendicular to the optical axis of the lens, and wherein the haptics portions do not extend beyond the shortest diameter of the lens.

The intraocular lenses of the present invention are particularly useful because the widest point of the IOL is the smallest of the diameters of the ovoid lens, and the haptics portions of the lens do not extend outwardly beyond this diameter thereby forming a lens of narrow profile allowing implantation through a smaller sized incision. The design and the substantially equal stiffness of the two loops or haptics in combination with the narrow profile of the lens make it particularly well suited to anterior capsulotomy.

The intraocular lens of the present invention may contain a UV absorbing amount of a UV absorber.

The following example is given to illustrate the production of an intraocular lens according to the present invention.

Example

A lens blank of a suitable material such as PMMA is wax mounted to an arbor. The posterior is lathe cut. The lens is remounted and the anterior is lathe cut. At this point, the optic is determined.

The profile is then milled on a wax mounted arbor to form the haptics. The lens is deblocked ie., the wax is dissolved and the lens floats free. The lens is then measured and cleaned. Thereafter, it is preferably tumble polished in glass beads in a polishing and water slurry.

The lens is then inspected to ensure conformity with the basic parameters of the lens, the power and resolution are determined and the lens is then cleaned, cosmetically inspected and packaged for sterilisation. Thereafter, the lens is sterilised, aerated and stored in a suitable sterile package.

The lens produced had the following parameters:

| Overall length | 14 mm |
|---|---|
| Lens size | 5 x 6 mm |
| Optic profile | convex-plano |
| Vaulting | 10° |
| Power | 10 dioptres |

**Claims**

1. An intraocular lens for implantation in the eye which comprises an ovoid lens having two haptics integral therewith, said haptics being of substantially equal stiffness, and wherein each haptic has an angle of from 0° to 15° as measured from the perpendicular to the optical axis of the lens.

2. An intraocular lens for implantation in the eye which comprises an ovoid lens having two haptics portions formed integrally therewith, which haptics portions are of substantially equal stiffness, and extend at an angle of from 0° to 15° as measured from the perpendicular to the optical axis of the lens, and wherein said haptics portions do not extend beyond the shortest diameter of the lens.

3. An intraocular lens according to claim 1 or claim 2 wherein the ovoid lens and integral haptics portions comprise poly methyl methacrylate.

4. An intraocular lens according to any one of the preceding claims in which the shortest diameter is less than 6mm.

5. An intraocular lens according to any one of the preceding claims wherein each haptic portion has a modified C configuration.

6. A intraocular lens according to any one of the preceding claims wherein each haptic portion is in the shape of an arc.

7. An intraocular lens according to claim 5 wherein the haptic comprises three portions of different length.

8. An intraocular lens according to any one of the preceding claims which contains a UV absorbing amount of a UV absorber.

9. An intraocular lens according to any one of the preceding claims wherein the haptics are of substantially the same size and shape.

10. An intraocular lens according to any one of the preceding claims wherein the ovoid lens has a first planar surface and a second convex surface.

11. An intraocular lens according to claim 10 whrein the convex surface forms the upper surface to the lens and the haptics have an angle of 10 - 15° as measured upwardly from the perpendicular to the optical axis of the lens.

12. An intraocular lens according to any one of the preceding claims wherein each haptic portion has an angle of about 10° as measured from the perpendicular to the optical axis of the lens

13. An intraocular lens for implantation in the eye which comprises ovoid lens having a first planar surface and a second convex surface and having two haptics integral therewith, said haptics being of substantial equal stiffness wherein each of said haptics comprises a first substantially columnar portion extending substantially tangentially from the lens periphery at a point where the shortest diameter of the lens intersects the lens periphery, a second arcuate portion extending from said first portion and a third arcuate portion extending from said second portion whose curvature is less than that of the second portion, wherein each haptic has an angle of from 0° to 15° as measured from the perpendicular to the optical axis of the lens, and wherein the haptics portions do not extend beyond the shortest diameter of the lens.

4

EP 0 404 478 A1

Neu eingereicht / Newly filed
Nouvellement déposé

# FIG. 1.

# FIG. 2.

| Category | Citation of document with indication, where appropriate, of relevant passages | Relevant to claim | CLASSIFICATION OF THE APPLICATION (Int Cl⁵) |
|---|---|---|---|
| X | US - A - 4 813 956 (GUPTA) * Totality; especially claims 1-8; fig. 4A,4B * | 1,3,8 | A 61 F 2/16 |
| A |  | 2,4,5, 6,13 |  |
| A | US - A - 4 725 277 (BISSONETTE) * Abstract; fig. 1 * | 1,13 |  |

**DOCUMENTS CONSIDERED TO BE RELEVANT**

TECHNICAL FIELDS SEARCHED (Int Cl⁵)

A 61 F

The present search report has been drawn up for all claims

| Place of search | Date of completion of the search | Examiner |
|---|---|---|
| VIENNA | 24-09-1990 | MIHATSEK |